# EUROPEAN PATENT SPECIFICATION

(11) **EP 2 432 482 B1**
(45) Date of publication and mention of the grant of the patent: **15.04.2015**
(21) Application number: 10723556.6
(22) Date of filing: 20.05.2010
(51) Int. Cl.: A61K 35/34

(54) **PHARMACEUTICAL COMPOSITION FOR THE TREATMENT OF HEART DISEASES.**
PHARMAZEUTISCHE ZUSAMMENSETZUNGEN ZUR BEHANDLUNG VON HERZERKRANKUNGEN
COMPOSITION PHARMACEUTIQUE POUR LE TRAITEMENT DE CARDIOPATHIES

(30) Priority: 20.05.2009 WO PCT/EP2009/056197
(43) Date of publication of application: 28.03.2012
(73) Proprietor: Cardio3 BioSciences S.A., 1435 Mont-Saint-Guibert (BE)
(72) Inventor: GAUSSIN, Vinciane, B-1970 Wezembeek-oppem (BE); GORDON-BERESFORD, Roland, B-1310 La Hulpe (BE); HOMSY, Christian, B-1150 Bruxelles (BE)
(74) Representative: Crease, Devanand John
(86) International application number: PCT/EP2010/057004
(87) International publication number: WO 2010/133686

(56) References cited:
- WO-A1-2008/109839
- WO-A2-2006/015127
- US-A1- 2008 019 944
- BEHFAR ATTA ET AL: "Cardiopoietic programming of embryonic stem cells for tumor-free heart repair" THE JOURNAL OF EXPERIMENTAL MEDICINE, ROCKEFELLER UNIVERSITY PRESS, UNITED STATES, vol. 204, no. 2, 1 February 2007 (2007-02-01), pages 405-420, XP002462528 ISSN: 0022-1007

## Description

### Field of the invention

The present invention relates to the treatment of heart disease disorders or predispositions of a disorder through delivery of a pharmaceutical composition to an individual in need. In particular, it discloses a pharmaceutical composition comprising cells committed to the generation of heart tissue and at least one pharmaceutically acceptable excipient, contained in a container in a way to allow cell survival and transportation to locations worldwide, convenient handling by staff for delivery to the recipient, wherein said cells are produced according to internationally recognized standards for pharmaceutical product manufacture.

### State of the art

Regenerative cell therapies are particularly relevant for diseases in which organs are compromised in such a way that tissue reconstruction is required, e.g. to restore the morphology as well as the function of a diseased organ, or when physiological repair mechanisms are impaired. The heart is a terminally differentiated organ and massive loss of cardiomyocytes such as in heart attack causes irreversible damages, hence the need for repair. In addition heart disease is a leading cause of mortality worldwide. Cell therapy for heart repair is a challenge of utmost importance.

Clinical experience with cell therapy has been based on adult stem cells delivered in an unaltered state. First generation biologics are naive human stem cells, identified as readily accessible cytotypes. It has been shown that a few individuals improve on delivery of naive human stem cells. The state of the art in the field of naive cell transplantation in the heart of humans was described inter alia in the review carried by Abdel-Latif A. et al. 'Adult bone marrow-derived cells for cardiac repair: a systematic review and meta-analysis.' Arch Intern Med. (2007) 167:989-997, and citations therein. In the hope of improving clinical outcome, the concept of second-generation stem cell therapies was developed and consists in improving the cardio-generative potential of naive stem cells prior to delivery to the patient.

In order to explore ways of improving cardio-generative potential of a cell, fundamental research first used mouse embryonic stem cells (hereinafter mESCs) to decipher complex signaling pathways involved during cardiac differentiation. This research led to the identification of cardiogenic substances which, in contact with a cell, improves the ability of said cell to differentiate into a cardiopoietic cell. The cardiopoietic cell is an intermediate cell phenotype where the cell is committed to the generation of heart tissue but not yet fully differentiated. Key milestones in basic research in the field of cardiac regeneration are disclosed in:
- Behfar et al. 'derivation of a cardiopoietic population from human mesenchymal stem yields progeny', Nature Clin. Pract., Cardiovasc. Med. (2006) 3: S78-S82,
- Behfar et al., 'Cardiopoietic programming of embryonic cells for tumor-free repairs', J. Exp. Med. (2007) 204: 405-420, and
- WO 2006/015127, US 2008/0019944 and WO 2009/151907, all to Mayo Foundation for Medical Education and Research, Terzic A. and Behfar A.

The above authors, applicant and inventors showed that the differentiation of mESCs into cells committed to the generation of heart tissue can be initiated when mESCs are cultured in contact with so-called 'cocktails' of cardiogenic substances, i.e. compositions containing cardiogenic factors in solution. As disclosed in WO2006/015127, when mESCs-derived cardiopoietic cells are delivered into chronically infarcted murine hearts, heart repair can be achieved. Thus, it has been made known that mESCs-derived cardiopoietic cells could show beneficial in regenerating heart tissue. However, the tumourigenic risk associated with ESCs raises safety issues to translate these basic findings for therapeutic use. Furthermore, the experiments were carried out in mice in the laboratory setting with mESC-derived cardiopoietic cells harvested from culture, suspended in culture medium in a laboratory tube, and promptly used thereafter within the same facility.

Adult stem cell therapy is considered to be devoid of tumourigenic risk. Identification of a source of stem cells with no documented tumourigenic risk and suitable for derivation into cardiopoietic cells was described in Behfar et al. 'Derivation of a cardiopoietic population from human mesenchymal stem yields progeny', Nature Clinical Practice, Cardiovascular Medicine (2006) 3:S78-S82. In US 2008/0019944 cardiopoietic cells obtained from mesenchymal stem cells are described.

The authors, applicant and inventors also disclosed that differentiation of human adult mesenchymal stem cells into cardiopoietic cells can be achieved using a cocktail of cardiogenic factors (WO 2009/151907).

The state of the art in the field of cell transplantation in the heart of humans was described inter alia in the review carried by Abdel-Latif A. et al. 'Adult bone marrow-derived cells for cardiac repair: a systematic review and meta-analysis. Arch Intern Med. (2007) 167:989-997, and citations therein. In another review by Behfar et al. Guided stem cell cardiopoietic: Discovery and translation' J. Mol. and Cell. Cardiology (2008) 45: 523-529, the concept of using cardiopoietic cells for heart regeneration was also discussed.

Moving from bench to bedside is usually a challenge for the pharmaceutical industry. In this case, this challenge has been particularly difficult to overcome because of the biological features of the cells committed to the generation of heart tissue and the absolute need to maintain these features till delivery to an individual when the individual is not present near the premises of the manufacturing premises.

The present invention solves this problem by disclosing a method for industrially producing a pharmaceutical composition containing cells committed to the generation of heart tissue and at least one pharmaceutically acceptable excipient according to internationally recognized standards for pharmaceutical product manufacture, a method for cell preservation and packaging that allows differed use whilst maintaining the features of cells committed to the generation of heart tissue, their survival, transportation to locations worldwide, convenient handling by staff for delivery to the recipient.

### Definitions

Within the frame of the present document, and unless indication of the contrary, the terms designated below between quotes have the following definitions.

'BMMSC' designates bone marrow mesenchymal stem cells. 'hBMMSC' designates such BMMSCs of human origin.

'Cardiopoietic cells' are an intermediate cell phenotype, i.e. committed to the generation of heart tissue but not yet fully differentiated. Cardiopoietic cells are characterized by nuclear translocation of Nkx2.5 and MEF2C, combined to the absence of sarcomeric proteins (Behfar et al. 'derivation of a cardiopoietic population from human mesenchymal stem yields progeny', Nature Clin. Pract., Cardiovasc. Med. (2006) 3: S78-S82*).* Cardiopoietic cells retain a proliferative capacity. Cardiopoietic cells can be derived from stems cells including for example, human adult mesenchymal stem cells, human embryonic stem cells (provided their production implies no human embryo destruction), embryonic-like stem cells, inducible pluripotent stem cells, or any other adapted source.

A 'cardiogenic cocktail' or 'cocktail' designates a composition containing at least two cardiogenic substances.

A 'cardiogenic substance' is a substance which, in contact with a cell, improves the ability of said cell to differentiate into a cardiopoietic cell.

'Confluence' designates the state in which cells have grown to maximum capacity within a certain amount of space. At this point contact with other cells causes them to inhibit growth.

The 'effective amount' means a sufficient amount of the pharmaceutical composition to provide the desired therapeutic or physiological effect or outcome. Such an effect or outcome includes the repair, maintenance, regeneration, augmentation of heart tissue or improvement of heart function. Undesirable effects are sometimes manifested along with the desired therapeutic effect; hence, a practitioner balances the potential benefits against the potential risks in determining what an appropriate 'effective amount' is. This amount may vary from subject to subject, depending for example on the subject age, general condition, genetic and epigenetic variability and the like, and the mode of administration. Thus, it may not be possible to specify an exact 'effective amount'. However, an appropriate 'effective amount' in any individual case may be determined by one of ordinary skill in the art before or during the administration procedure of the pharmaceutical composition, for example by delivering the highest amount possible without undesirable effect during delivery of the pharmaceutical composition.

'Excipient' is an inactive substance used as a carrier for the active ingredients of a medication. In many cases, an "active" substance may not be easily administered and absorbed by the human body; in such cases the substance in question may be dissolved into or mixed with an excipient. In addition to their use in the single-dosage quantity, excipients can be used in the manufacturing process to aid in the handling of the active substance concerned. Depending on the route of administration, and form of medication, different excipients may be used. To stabilize the active ingredient, excipients are added, ensuring that the active ingredient stays "active", and, just as importantly, stable for a sufficiently long period of time that the shelf-life of the product makes it competitive with other products.

'Proliferative capacity' designates within the frame of this document, an increase of cell number.

'Viability' means within the frame of this document the feature for cells of not taking up the trypan blue dye, thereby demonstrating cell membrane integrity.

The terms 'subject', 'recipient' and 'patient' are used interchangeably herein and refer unless explicitly stated to any human or mammal in need of treatment for a cardiac disease or disorder with the hereby disclosed pharmaceutical composition. Subjects also include those at risk of having such a cardiac disease or disorder.

As used in the subject specification, the singular forms 'a', 'an' and 'the' include plural aspects unless the context clearly dictates otherwise. Thus, for example, reference to 'a stem cell' includes a single cell, as well as two or more cells; reference to 'an agent' or 'a reagent' includes a single agent or reagent, as well as two or more agents or reagents; reference to 'the invention' or 'an invention' includes single or multiple aspects of an invention; and so forth.

Unless otherwise defined, all technical and scientific terms used herein have the same meaning as commonly understood by one of ordinary skill in the art to which this invention pertains. Although methods and materials similar or equivalent to those described herein can be used in the practice or testing of the present invention, suitable methods and materials are described below.

### Summary of the invention

In the following detailed description, numerous details are set forth in order to provide a thorough understanding of the present claimed subject matter. However, it will be understood by those skilled in the art that the claimed subject matter may be practiced without these specific details. In other instances, well-known methods, procedures, components, and alike have not been described in detail so as to not obscure the claimed subject matter.

The present invention relates to a pharmaceutical composition comprising cells committed to the generation of heart tissue and at least one pharmaceutically acceptable excipient. Cells committed to the generation of heart tissue are preferably produced according to internationally recognized standards for pharmaceutical product manufacture. Most preferably, said pharmaceutically acceptable excipient is a preservation solution. Preferably, the preservation solution may be chosen in the group comprising preservation solutions which allow cryopreservation at temperatures between -196°C and 0°C and preservation solutions which allow preservation at temperatures between 0°C and +40°C. Preferably, the preservation solution is a preservation solution which may contain ions, pH buffers, impermeants, colloids and metabolites, dimethylsulfoxide (DMSO), glycerol, sucrose, serum albumin, trehalose, or any combination thereof. Preferably, ions are selected from the group consisting of Na⁺, K⁺, Ca²⁺, Mg²⁺, Cl⁻ and combinations of said ions. Preferably, pH buffers are selected from the group consisting in H₂PO₄⁻, HCO₃⁻, (4-(2-hydroxyethyl)-1-piperazineethanesulfonic acid) (HEPES) and mixtures thereof. Preferably, impermeants are selected from the group consisting of lactobionate, sucrose, mannitol, glucose and combinations thereof. Preferably, the colloid is Dextran-40. Preferably, the metabolites are selected from the group consisting of adenosine, glutathione, and combinations thereof. Preferably, said at least one pharmaceutically acceptable excipient further comprises at least one component selected from the group consisting of growth factors, cytokines, proteins involved in organogenesis signalling, pharmaceuticals, platelet lysate, serum, isotopes, means for tracing cells *in vivo,* diluents, lubricants, matrix or scaffold materials, and combinations thereof.

Preferably, cells committed to the generation of heart tissue may be stem cells or cells committed to the generation of heart tissue derived from stem cells (provided their production implies no human embryo destruction). Preferably, stem cells are selected in the group consisting of adult stem cells, embryonic stem cells (provided their production implies no human embryo destruction), induced pluripotent stem cells (IPS), marrow-isolated adult multilineage inducible cells (MIAMI), resident cardiac stem cells, vegetal stem cells, or any combination thereof. Preferably, stem cells are mesenchymal stem cells harvested from a suitable tissue source selected in the group consisting of bone marrow, adipose tissue, umbilical cord blood, amniotic fluid, menstrual fluid, blood. Preferably, cells committed to the generation of heart tissue are mammalian cells. Preferably, mammalian cells are selected in the group consisting of humans, cats, dogs, pigs, horses, mice, rats, hamsters and other mammals. Preferably, said cells are autologous cells, allogeneic cells, xenogenic cells, or any combination thereof.

Preferably, cells committed to the generation of heart tissue are cardiopoietic cells. Preferably, cardiopoietic cells can be derived from stems cells including for example, from human adult mesenchymal stem cells, human embryonic stem cells (provided their production implies no human embryo destruction), embryonic-like stem cells, inducible pluripotent stem cells, cardiac resident stem cells or any other adapted source or combination thereof.

Preferably, said composition contains no detectable non-cardiopoietic cells, including cardiomyocytes, hematopoietic cells, endothelial progenitor cells, adipoblasts, adipocytes, chondroblasts, chondrocytes, osteoblasts osteocytes, neuroblasts and neurocytes. Preferably, the contents of total non-cardiopoietic cells is between 0% and 50% of the total number of cells, preferably between 0% and 15% of the total number of cells. Preferably, the contents of each category of non-cardiopoietic cells is between 0% and 50% of the total number of cells, preferably between 0% and 15% of the total number of cells.

Further described herein is a pharmaceutical composition for use for the treatment of ischemic cardiomyopathy, acute myocardial infarction, chronic myocardial infarction, heart failure of non-ischemic origin, heart failure of ischemic origin, congenital cardiomyopathy, or combination thereof.

The invention also relates to a process for the manufacture of a pharmaceutical composition which comprises the following steps:
- obtaining cells from which cells committed to the generation of heart tissue can derive;
- culturing said cells in conditions which allow to obtain cells committed to the generation of heart tissue;
- harvesting said committed cells;
- adding to said committed cells at least one pharmaceutically acceptable excipient.

Preferably, the process of the invention is conducted according to internationally recognized standards for pharmaceutical product manufacture, which comprises taking samples at any step of the process for the purpose of performing quality control operations. Most preferably, the process of the invention is conducted according to internationally recognized standards for pharmaceutical product manufacture, which comprises taking samples at the last step of cell culture for the purpose of performing quality control of the active substance.

Preferably, quality control criteria of the active substance comprise at least one test selected from the group consisting of identity test, homogeneity test, purity test and combinations thereof. Preferably, when cells committed to the generation of heart tissue are cardiopoietic cells, identity of said cardiopoietic cells corresponds to an observed increase in expression level of at least one gene in the group consisting of Nkx2.5, Tbx5, MEF2C, GATA4, GATA6, Mesp1, FOG1, FOG2, Flk1 and homologues thereof. Preferably, the increase of gene expression is a minimum 2-fold as determined by qPCR method, as compared to a reference. Preferably, when cells committed to the generation of heart tissue are cardiopoietic cells, identity of said cardiopoietic cells is considered fulfilled by the observed presence of at least one polypeptide species chosen from the group consisting of Nkx2.5, Tbx5, MEF2C, GATA4, GATA6, Mesp1, FOG1, FOG2, Flk1 and homologues thereof, as compared to a reference, and either Nkx2.5 or MEF2C or both being further translocated to the nuclei of the cardiopoietic cells. Preferably, the observed presence is shown by immuno-labelling with at least one antibody in the group consisting of anti-Nkx2.5, anti-Tbx5, anti-MEF2C, anti-GATA4, anti-GATA6, anti-Mesp1, anti-FOG1, anti-FOG2, anti-Flk1, and homologues thereof. Preferably, homogeneity is fulfilled when at least 50% in a given sample are cardiopoietic cells. Preferably, homogeneity is fulfilled when at least 50%, most preferably at least 85%, in a given sample are cardiopoietic cells. Preferably, presence of mesenchymal stem cells amongst harvested cells is shown by positive immuno-labelling with an antibody against a surface marker selected in the group consisting of CD105, CD90, CD133, CD105, CD166, CD29, and CD44, and lack of detectable immune-labelling with an antibody against a surface marker selected in the group consisting of CD14, CD34, and CD45. Preferably, purity is not fulfilled with increased in expression level of the CD34, FABP4, osteocalcin, nestin, Sox9, and MYH7 genes and homologues thereof two-fold or greater when compared to a reference. Preferably, the increase of gene expression is determined by qPCR method, as compared to a reference. Preferably, purity is not fulfilled with increased number of non-cardiopoietic cells, including cardiomyocytes, hematopoietic cells, endothelial progenitor cells, adipoblasts, adipocytes, chondroblasts, chondrocytes, osteoblasts osteocytes, neuroblasts and neurocytes, as shown by immuno-labelling. Preferably, said reference consists of non-cardiopoietic cells. Preferably, said reference consists of cells cultured in the absence of any cardiogenic substance. Preferably, culturing conditions include using a bioreactor which comprises immobilising or encapsulating said cells on particles or on a matrix and passing cell culture media through the bed of particles or matrix.

Further described herein is a method for the treatment of heart disease disorders or predisposition of a disorder wherein the pharmaceutical is delivered into an individual in an effective amount. Preferably, the individual shows an insufficiency of the cardiovascular system. Preferably, the individual suffers from ischemic cardiomyopathy, myocardial infarction, heart failure of ischemic origin, or heart failure of non-ischemic origin, congenital cardiomyopathy, or combination thereof.

Preferably, the pharmaceutical composition is delivered using a route of administration selected in the group consisting of intra-myocardial, intra-cardiac, intracoronary, intra-muscular, sub-cutaneous, intra-peritoneal, *in utero,* parenteral, or systemic. Preferably, the pharmaceutical composition is injected intramyocardially using a catheter, a syringe, or combination thereof.

Cells from which cells committed to the generation of heart tissue can derive may be put into contact with at least one cardiogenic substance, which may be selected from the group consisting of TGF-β1, TGF-β2, TNF-α, BMP-1, BMP-2, BMP-4, BMP-6, FGF-2, FGF-4, FGF-5, FGF-12, FGF-13, FGF-15, FGF-20, leukaemia inhibitory factor (LIF), VEGF-A, VEGF-C, insulin-like growth factor 1 (IGF-1), interleukin 6 (IL-6), Activin A, α thrombin, retinoic acid, cardiotrophin 1, cardiogenol C, and combinations thereof.

A great number of cardiogenic cocktails may be used. The list given below is not limitative. One may use for instance a cocktail of cardiogenic substances that comprises TGFβ-1, BMP4, α-thrombin, a compound selected from the group consisting of Cardiotrophin and IL-6, and a compound selected from the group consisting of Cardiogenol C and retinoic acid. Another cocktail may comprise TGFβ-1, BMP4, α-thrombin, Cardiotrophin and Cardiogenol C. Still another cocktail may comprise at least one compound selected from the group consisting of FGF-2, IGF-1, Activin-A, TNF-α, FGF-4, LIF, VEGF-A and combinations thereof. They may also comprise FGF-2, IGF-1 and Activin-A. Other preferred cocktails comprise Activin-A, FGF-2, IL-6, IGF-1 and retinoic acid. Other cocktails can lack at least one compound chosen in the group consisting of TNF-α, FGF-4, LIF, and VEGF-A.

When one of the following compounds is present in a cocktail, it may be present in an amount of between 1 and 5 ng of said TGFβ-1 per ml, between 1 and 10 ng of said BMP4 per ml, between 0.5 and 5 ng of said Cardiotrophin per ml, between 0.5 and 5 units of said α-thrombin per ml, and between 50 and 500 nM of said Cardiogenol C, between 1 and 10 ng of said FGF-2 per ml, between 10 and 100 ng of said IGF-1 per ml, between 1 and 50 ng of said Activin-A per ml, between 1 and 50 ng of said TNF-α per ml, between 1 and 20 ng of said FGF-4 per ml, between 10 and 100 ng of said IL-6 per ml, between 1 and 10 units of said LIF per ml, between 1 and 50 ng of said VEGF-A per ml, between 0.1 and 1.0 µM of said retinoic acid per ml.

A particular cocktail type comprises recombinant TGFβ-1(2.5 ng/ml), BMP4 (5 ng/ml), Cardiotrophin (1 ng/ml), Cardiogenol C (100 µM), used in a combinatorial fashion. Particularly preferred cocktails comprises such compounds and further comprise α-thrombin, (1 U/ml), FGF-2 (10 ng/ml), IGF-1 (50 ng/ml) and Activin-A (5 ng/ml).

Other preferred cocktails comprise recombinant TGFβ-1 (2.5 ng/ml), BMP4 (5 ng/ml), Activin-A (5 ng/ml), FGF-2 (10 ng/ml), IL-6 (100 ng/ml), Factor-Ila (hα-thrombin, 1 U/ml), IGF-1 (50 ng/ml), and retinoic acid (1 µM) used in a combinatorial fashion.

The cocktail may be diluted in a medium containing compounds selected from the group consisting of foetal calf serum, human serum, platelet lysate, platelet-derived growth factor, and mixtures thereof and compounds selected. [0041] The present invention also relates to a kit for the administration of a pharmaceutical composition which comprises a container containing said pharmaceutical composition. Preferably said container is a biocompatible container that allows cell survival and transportation worldwide, convenient handling by staff for delivery to the recipient. Preferably said container is hermetically closed. Preferably said container is compatible with the excipient and conditions of preservation. Preferably said container is a closed glass container. Preferably said container has a piercable septum cap. Preferably said piercable septum cap allows a vial adapter that includes a luer activated valve to draw fluid from the container. Preferably said piercable septum cap can be accessed with a needle. Preferably said pharmaceutical composition is stored in a hermetically closed container suitable for cryopreservation. Preferably, shelf-life of pharmaceutical composition in said container is at least 48 hours, preferably 72 hours. Preferably, said kit further comprises at least one catheter. Preferably, said kit further comprises at least one syringe.

### Brief Description of the Drawing

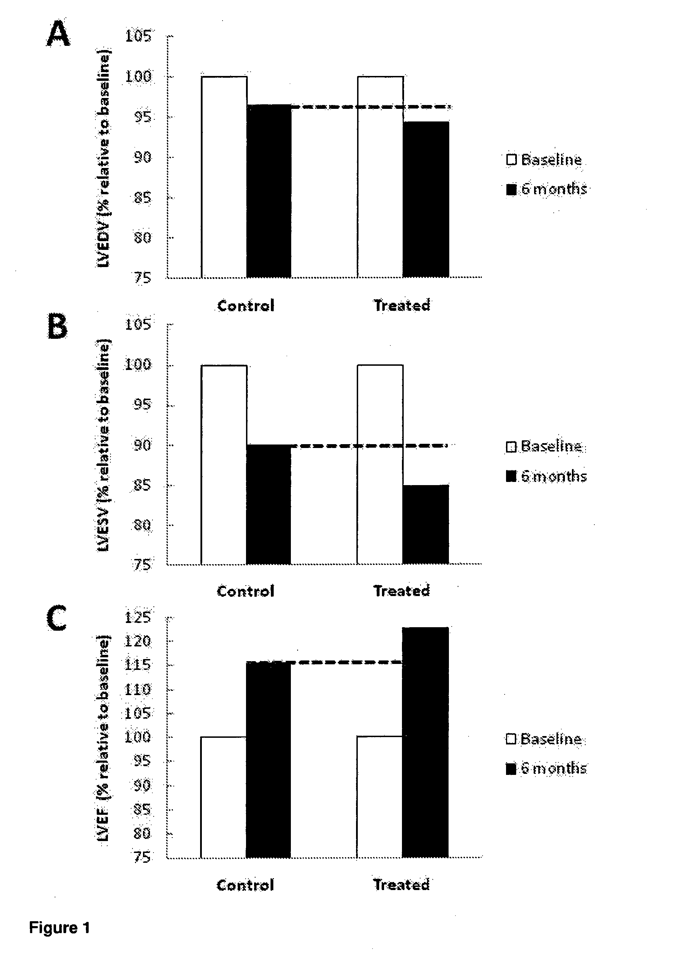

Fig 1. shows Left Ventricular (LV) End Diastolic Volume (LVEDV, panel A), LV End Systolic Volume (LVESV, panel B), and LV Ejection Fraction (LVEF, panel C), that were measured at baseline and 6 months thereafter in subjects randomly assigned to control (n=8) and treated (n=9) groups. Results were normalized to baseline.

### Detailed description of the invention

### Example

Start of manufacture: Human bone marrow sample having been harvested from the iliac crest of a patient and meeting minimum quality criteria are cultured at 37°C/5% CO₂ incubators in 175-cm² flasks to purify BMMSCs. Minimum quality criteria include negative serology test of the donor (at least HIV 1/2, syphilis, HBV, HCV), bone marrow transportation temperature control between collection site and manufacturing site, total volume, recording the presence and volume of blood clots, and the absence of bacterial contamination. After 24 hours, bone marrow and cellular debris are carefully discarded from the flasks. Adherent BMMSCs are washed with phosphate buffer saline (PBS), culture medium is added, and culture is resumed until initial passage P0 with change of medium every four to six days.

P0 - Initial expansion from colonies to cell layer: An initial passage (P0) is performed to dissociate colonies and to allow them to expand and form a monolayer. Cells are seeded at a one-to-one ratio in 175-cm² flasks and allowed to expand and form a monolayer for up to 6 days. Confluence determines timing for the next passage. This step may be skipped if BMMSCs spontaneously form a monolayer with no detectable colonies. The process continues with similar passages numbered 'P0' followed by a sequential letter until a minimum of 50x10⁶ cells is obtained. Parameters such as cell density at seeding and confluence-triggered passages are defined. It is the number of cells obtained at a passage that determines the container size that should be used for seeding in order to optimize yield and to avoid contact inhibition. In-process control testing in the P0 phase includes cell number and percent viability.

P1 - Start of cardiogenic cocktail treatment: Cells are cultured for 5 days in culture medium and a cardiogenic cocktail. For instance, cardiogenic cocktails such as described in WO2006/015127, WO2009/151907 and Behfar et al. 'Derivation of a cardiopoietic population from human mesenchymal stem yields progeny', Nature Clinical Practice, Cardiovascular Medicine, March 2006 vol. 3 supplement 1, pages S78-S82) may be used.

P2 - End of cardiogenic cocktail treatment: The medium containing the cardiogenic cocktail is discarded. The culture now contains cardiopoietic cells. The culture is passaged and seeded in new containers with culture medium to allow for additional expansion phases as needed.

P3 - Expansion and harvest: The following passages are numbered 'P3' followed by a sequential letter. Cells are passaged when optimal confluence is reached, and repeated until the number of cells obtained is between 600x10⁶ and 1200x10⁶ cells. When this criterion is met, the cells are harvested. This step involves a final trypsinization followed by washing and concentration steps by centrifugation. The final wash is performed in a cell preservation solution. Suitably, the preservation solution employed may be similar to standard organ and biological tissue preservation aqueous cold storage solutions such as HypoThermosol-FRS^{®} from BioLifeSolutions (Bothell, Wash).

The cell concentrate is then transferred into a biocompatible container (in this example glass bottle of Type I, Ph. Eur.) and preservation solution added to reach a total volume of 10 ml and a cellular concentration in the range of 60x10⁶ to 120x10⁶ cells/ml. This finalizes the manufacture of the pharmaceutical composition.

The pharmaceutical composition release criteria typically include cellular identity, homogeneity and purity that are combined with manufacture parameters that confirm the absence of adventitious contamination (asepsis, low level of endotoxin, and mycoplasma not added by the process).

It is worth noting that without any prejudice to present invention, other methods of production of cardiopoietic cells could be envisaged instead of the use of cardiogenic substances.

In this example, the preservation medium is HypoThermosol-FRS^{®} from BioLifeSolutions (Bothell, Wash). HypoThermosol-FRS contains ions (100 mM Na+, 42.5 mM K+, 0.05 mM Ca2+, 5 mM Mg2+, 17.1 mM CI-); pH buffers (10 mM H2PO4-, 5 mM HCO3-, 25 mM HEPES); impermeants to counteract cell swelling (100 mM lactobionate, 20 mM sucrose, 20 mM mannitol); colloid (6% Dextran-40); and metabolites (5 mM glucose, 2 mM adenosine, 3 mM glutathione).

Preservation of the pharmaceutical composition may also be achieved according to the invention by cryopreservation in dimethylsulfoxide (DMSO). This offers the advantage of allowing even longer shelf-life (one week or more) provided transportation on dry ice, with proper control of transportation temperature.

According to the embodiment of the pharmaceutical composition of the present invention wherein the excipient is HypoThermosol-FRS, the shelf-life of the pharmaceutical product a container is at least 72 hours. It is particularly remarkable to observe that a total number of 1200x10⁶ cells have a volume of about 8 millilitres, whereas the maximum desirable volume for intramyocardial injection is about 10 millilitres. This means that the quantity of HypoThermosol-FRS is small vis-à-vis the cellular volume and will be in the range of only a few millilitres.

It has surprisingly been observed that even such a small quantity of HypoThermosol-FRS is sufficient to maintain such an important shelf-life of 72 hours for the pharmaceutical composition. This provides for sufficient time to confirm all release criteria are met, to ship the pharmaceutical composition anywhere in the world, and to deliver it to the recipient.

Pharmaceutical composition release criteria - Identity: Cardiopoietic cells are characterized by positive expression and, when applicable, nuclear translocation of several markers of early cardiac differentiation, including Nkx2.5, MEF2C and GATA-4. Positive identity of cardiopoietic cells contained in the pharmaceutical composition is represented by a minimum 2-fold increase in expression level for MEF2C and/or Tbx5 compared to the reference standard, measured by real-time quantitative RT-PCR (qPCR) and maintenance thereof during shelf-life.

In this preferred embodiment, Table 1, Table 2 and Table 3 below show the expression of early cardiac differentiation markers is maintained for as long as 14 days after the pharmaceutical composition is stored in its final container, and that viability and proliferation of such cells is maintained for at least 5 days. This demonstrates the unique capacity of the manufacturing process hereby described to obtain and maintain the identity of the cells exposed to the cardiogenic substances in a state suitable for their intended use.

**Table 1 Identity (qPCR) at 100 million total viable cells per ml.**

| Day | Batch 1 | | Batch 2 | |
|---|---|---|---|---|
| | MEF2C | Tbx5 | MEF2C | Tbx5 |
| 0 | 2,5 | 2,6 | 3,0 | 2,0 |
| 1 | 2,1 | 2,4 | 2,2 | 1,7 |
| 2 | 2,5 | 2,6 | 2,6 | 2,0 |
| 3 | 2,2 | 2,1 | 2,9 | 1,9 |
| 4 | 2,3 | 2,5 | 2,3 | 3,0 |
| 5 | 2,0 | 1,8 | 2,5 | 2,0 |
| 6 | 2,3 | 1,0 | 2,7 | 1,7 |
| 10 | 2,1 | 2,0 | 3,0 | 3,1 |
| 14 | 2,3 | 1,5 | 3,5 | 2,6 |

**Table 2: Viability of the pharmaceutical composition at cellular concentration of 100 million total viable cells/ml.**

| Day | Batch 1 | Batch 2 |
|---|---|---|
| 0 | 96 | 96 |
| 1 | 88 | 95 |
| 2 | 92 | 96 |
| 3 | 87 | 93 |
| 4 | 91 | 96 |
| 5 | 87 | 91 |

**Table 3: Proliferative capacity of the pharmaceutical composition at cellular concentration of 100 million total viable cells/ml.**

| Day | Batch 1 | Batch 2 |
|---|---|---|
| 1 | 444 | 456 |
| 2 | 297 | 481 |
| 3 | 417 | 333 |
| 4 | 306 | 417 |
| 5 | 303 | 722 |

Furthermore, Table 4 and Table 5 show that the pharmaceutical composition is not limited to a singly defined cell concentration yield. Indeed, maintenance of cell viability and proliferative capacity at different cellular concentrations are distinguishing features of the pharmaceutical composition hereby described.

**Table 4 Influence of cell concentration on percent viability**

| Day | Batch 1 (in Mio cells/ml) | | | Batch 2 (in Mio cells/ml) | |
|---|---|---|---|---|---|
| | 80 | 100 | 110 | 100 | 120 |
| 0 | 96 | 96 | 96 | 96 | 96 |
| 1 | 90 | 88 | 91 | 95 | 94 |
| 2 | 90 | 92 | 90 | 96 | 87 |
| 3 | 90 | 87 | 88 | 93 | 93 |
| 4 | 94 | 91 | 90 | 96 | 96 |
| 5 | 90 | 87 | 88 | 91 | 94 |

**Table 5 Influence of cell concentration on proliferative capacity**

| Day | Batch 1 (in Mio cells/ml) | | | Batch 2 (in Mio cells/ml) | |
|---|---|---|---|---|---|
| | 80 | 100 | 80 | 100 | 80 |
| 1 | 375 | 444 | 333 | 456 | 444 |
| 2 | 174* | 297 | 314 | 481 | 425 |
| 3 | 694 | 417 | 500 | 333 | 333 |
| 4 | 278 | 306 | 292 | 417 | 444 |
| 5 | 256 | 303 | 214 | 722 | 583 |

| | | | | | |
|---|---|---|---|---|---|
| The value obtained for batch 1 at day 2 for 80 million cells per ml identified with (*) is considered an experimental error. | | | | | |

Pharmaceutical composition release criteria - Homogeneity: In order to determine the percentage of cardiopoietic cells in the pharmaceutical composition hereby described in this preferred embodiment, dual-immunolabelling is performed on an aliquot of cells with antibodies against MEF2C and CD105, followed by nuclear staining using DAPI. The goal is to determine the percentage of cardiopoietic cells (nuclear staining for MEF2C) and mesenchymal stem cells (CD105-positive), with the total number of counted cells given by the number of DAPI-stained nuclei. Analysis of patient-derived cardiopoietic cells that passed the identity test by qPCR (MEF2C:2.8±0.6 fold increase, Tbx5: 2.2±0.6 fold increase) shows that 96±2% of the cells are cardiopoietic. In addition, 100% of the counted cells are CD105-positive. Taken together with the lack of increase in the level of expression of CD34, a marker for hematopoietic cells and endothelial progenitor cells (see paragraph on 'Purity'), this indicates that 100% of the cells are mesenchymal stem cells or derived from mesenchymal stem cells.

Pharmaceutical composition release criteria - Purity: The purity test performed according to the preferred embodiment of this invention aim at determining that cell types different from cardiopoietic cells and BMMSCs are absent from the pharmaceutical composition. A method of choice to address purity criteria is qPCR. The approach taken to develop the qPCR method for purity testing included the identification of suitable markers, design of proprietary primer and probe sets, analysis of amplification curves and melting peaks, and identification of commercially available positive control RNAs. Absence of the hematopoietic phenotype and of the endothelial progenitor phenotype both normally present in bone marrow is determined by the absence of detectable levels of CD34-expressing cells in the pharmaceutical composition. Absence of adipoblasts, chondroblasts, osteoblasts or neuroblasts is determined in the pharmaceutical composition by the absence of detectable levels of FABP4-expressing cells, Sox9-expressing cells, osteocalcin-expressing cells and nestin-expressing cells, respectively. Exclusion of mature cardiomyocytes is evaluated by the absence of detectable levels of MYH7-expressing cells in the pharmaceutical composition.

The herein described preferred embodiment for the pharmaceutical composition of the invention was injected endocardially in a human with heart failure of ischemic origin in an efficient amount of 1200x10⁶ cells into the in the border zone of the non-viable myocardium using a dedicated catheter. Satisfactory results were obtained.

The feasibility, safety and efficacy of a pharmaceutical composition containing cardiopoietic cells, according to the invention, was evaluated in a prospective, randomized, open, sequential parallel two-armed, multi-centre clinical trial.

Subjects presenting with chronic heart failure secondary to ischemic cardiomyopathy were randomly assigned to the control or treated group. The control group received optimal standard of care. The treated group received the pharmaceutical composition containing cardiopoietic cells in addition to optimal standard of care.

The pharmaceutical composition containing cardiopoietic cells was injected endoventricularly in the border zone of the infracted area using the MyoStar® Injection Catheter (Biologics Delivery Systems, California, USA). In a single injection procedure, up to 1,2 x 10⁹ cells were injected in up to twenty injection sites surrounding the infarcted area.

Two-dimensional echocardiographic evaluation of left ventricular (LV) function was performed in 17 subjects enrolled in this trial (9 treated, 8 control) at baseline and at 6 months thereafter. Cardiac function was assessed by measuring the change in LV End Diastolic Volume (LVEDV), LV End Systolic Volume (LVESV), and LV Ejection Fraction (LVEF) from baseline to 6 month post-cell injection. A trend was observed for these three important parameters in the treated group in favour of a positive effect of the pharmaceutical composition on the cardiac function (Figure 1). Physician skilled in the art would recognize the important prognostic and therapeutic implications these results carry.

### Other embodiments

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

In particular, the pharmaceutical composition described herein, including its storage conditions and shelf-life, is only a preferred embodiment that depicts use of autologous cardiopoietic cells derived from bone marrow mesenchymal stem cells (BMMSCs), i.e. a pharmaceutical composition prepared from BMMSCs to be used in the same individual the BMMSCs were collected from. The scope the present invention is not limited to autologous BMMSCs and includes use of any stem cells, irrespective of the source. Cells which allow to obtain cells committed to the generation of heart tissue (herein after in this paragraph 'original cells') may be allogeneic and xenogenic. Original cells may also be obtained by other means than fresh bone marrow procurement. Original cells may be embryonic stems cells provided that their procurement does not involve destruction of human embryos. Original cells can be embryonic-like stem cells such as Induced Pluripotent Stem (IPS) cells obtained by any means including transfection, cellular reprogramming or other method which renders IPS free of exogenous genes. Original cells can also be Marrow-Isolated Adult Multilineage Inducible (MIAMI) cells, resident cardiac stem cells, vegetal stem cells, or any combination thereof.

In a further embodiment, the pharmaceutical composition described herein may include additional components for example cardiogenic substances, growth factors such as fibroblast growth factors, placental growth factor or vascular endothelial growth factor, cytokines, or proteins involved in organogenesis signalling, molecular constructs, non-cardiopoietic cells altered ex vivo, pharmaceuticals, platelet lysate, scaffold materials such as collagen, laminin, or any other extracellular matrix proteins.

In a further embodiment, the kit described herein may include a catheter according to PCT/EP2010/055869, TW099113613, US 61/312371, BE2009/0271, PCT/EP2010/055856, TW099113627, or BE2009/0272.

In a further embodiment, the kit described herein may include additional components for instance bags or media suitable for refrigeration, freeze, cryopreservation, lyophilisation, vitrification, thawing, rehydration, washing, sorting, concentration, filtering, lyophilisation, centrifugation, resuspension, sampling, or aliquoting the pharmaceutical composition.

In a further embodiment, the kit described herein may include thermomonitoring, anti-tamper device, or radio-frequency identification device.

It is also to be understood that the terminology used herein is for the purpose of describing particular embodiments only and is not intended to be limiting.

## Claims

1. A pharmaceutical composition comprising cells committed to the generation of heart tissue, wherein said cells show:
i. an observed increase in expression level as compared to a reference of MEF2C and optionally at least one gene further selected from the group consisting of Nkx2.5, Tbx5, GATA4, GATA6, Mesp1, FOG1 FOG2, FIk1; and/or
ii. an observed presence of at least one polypeptide species chosen from the group consisting of Nkx2.5, Tbx5, GATA4, GATA6, Mesp1, FOG1 FOG2, Flk1, as compared to a reference and nuclear translocation to the nuclei of the cell of one or more of Nkx2.5 and MEF2C;
and at least one pharmaceutically acceptable excipient, wherein said at least one pharmaceutically acceptable excipient is a preservation solution.

2. The pharmaceutical composition of claim 1, wherein the observed increase in expression level is a minimum of 2-fold as determined by qPCR, as compared to a reference.

3. A pharmaceutical composition according to any of the previous claims, wherein the preservation solution is chosen in the group comprising preservation solutions which allow cryopreservation at temperatures between -196°C and 0°C and preservation solutions which allow preservation at temperatures between 0°C and +40°C.

4. A pharmaceutical composition according to any of the previous claims, wherein the preservation solution is a preservation solution which may contain ions (Na⁺, K⁺, Ca²⁺, Mg²⁺, Cl⁻ and combinations thereof), pH buffers (H₂PO₄⁻, HCO₃⁻, (4-(2-hydroxyethyl)-1-piperazineethanesulfonic acid) (HEPES) and mixtures thereof), impermeants (lactobionate, sucrose, mannitol, glucose and combinations thereof), colloids (Dextran-40), and metabolites (adenosine, glutathione, and combinations thereof), dimethylsulfoxide (DMSO), glycerol, sucrose, serum albumin, trehalose, or any combination thereof.

5. A pharmaceutical composition according to any of the preceding claims, wherein said at least one pharmaceutically acceptable excipient comprises at least one component selected from the group consisting of growth factors, cytokines, proteins involved in organogenesis signalling, pharmaceuticals, platelet lysate, serum, isotopes, means for tracing cells *in vivo,* diluents, lubricants, matrix or scaffold materials, and combinations thereof.

6. A pharmaceutical composition according to any of the preceding claims, wherein said cells committed to the generation of heart tissue are stem cells (provided their production implies no human embryo destruction).

7. A pharmaceutical composition according to any of the preceding claims, wherein said cells committed to the generation of heart tissue are derived from stem cells (provided their production implies no human embryo destruction).

8. A pharmaceutical composition according to claims 6 or 7 wherein said stem cells are selected in the group consisting of adult stem, including mesenchymal stem cells; embryonic stem cells (provided their production implies no human embryo destruction); induced pluripotent stem cells (IPS); marrow-isolated adult multilineage inducible cells (MIAMI); resident cardiac stem cells; vegetal stem cells; and combinations thereof.

9. A pharmaceutical composition according to any of the preceding claims, wherein said cells committed to the generation of heart tissue are mammalian cells.

10. A pharmaceutical composition according to any of the preceding claims, wherein said cells committed to the generation of heart tissue are cardiopoietic cells which can be derived from human adult mesenchymal stem cells, human embryonic stem cells (provided their production implies no human embryo destruction), embryonic-like stem cells, inducible pluripotent stem cells, cardiac resident stem cells or any other adapted source or combination thereof.

11. A pharmaceutical composition according to claim 10 wherein between 0% and 50% of the total number of cells, preferably between 0% and 15% of the total number of cells are non-cardiopoietic cells, including but not limited to cardiomyocytes, hematopoietic cells, endothelial progenitor cells, adipoblasts, adipocytes, chondroblasts, chondrocytes, osteoblasts osteocytes, neuroblasts and neurocytes.

12. Pharmaceutical composition according to any one of claims 1 to 11 for use for the treatment of ischemic cardiomyopathy, acute myocardial infarction, chronic myocardial infarction, heart failure of non-ischemic origin, heart failure of ischemic origin, congenital cardiomyopathy, or combination thereof.

13. Process for the manufacture of a pharmaceutical composition according to any of the preceding claims comprising the following steps:
- obtaining cells from which cells committed to the generation of heart tissue can be derived;
- culturing said cells in conditions which allow to obtain cells committed to the generation of heart tissue;
- harvesting said committed cells;
- adding to said committed cells at least one pharmaceutically acceptable excipient wherein said at least one pharmaceutically acceptable excipient is a preservation solution.

14. Process according to claim 13 wherein said cells from which cells committed to the generation of heart tissue can derive are put into contact with a cardiogenic composition composed of one or more cardiogenic substances chosen amongst the group consisting of: Activin A, α-thrombin, angiopoietin, Bone morphogenetic proteins (BMP) such as BMP-1, BMP-2, BMP-4, BMP-5, BMP-6, Cardiotrophin 1, Cardiogenol C, Epidermal growth factor (EGF), Erythropoietin (EPO), Fibroblast growth factors (FGF) such as FGF-1, FGF-2, FGF-4, FGF-5, FGF-12, FGF-13, FGF-15, FGF-20, Granulocyte-colony stimulating factor (G-CSF), Granulocyte-macrophage colony stimulating factor (GM-CSF), Growth differentiation factor-9 (GDF-9), Hepatocyte growth factor (HGF), Insulin-like growth factor (IGF) such as IGF-1, IGF-2, Myostatin (GDF-8), Neurotrophins such as NT-3, NT-4, NT-1 and Nerve growth factor (NGF), Platelet-derived growth factor (PDGF) such as PDGF-beta, PDGF-AA, PDGF-BB, Thrombopoietin (TPO), TGF- (Transforming growth factor alpha), Transforming growth factors β (TGF- β) such as TGF-β1, TGF-β2, TGF-β3, VEGF (Vascular endothelial growth factor) such as VEGF-A, VEGF-C, TNF-α, Leukemia Inhibitory Factor (LIF), interleukin 6 (IL-6), retinoic acid, C SDF-1 (stromal cell-derived factor-1), BDNF (brain-derived neurotrophic factor), Periostin, Angiotensin II, Flt3 Ligand, Glial-Derived Neurotrophic Factor, Insulin-Like Growth Factor Binding Protein-3, Insulin-Like Growth Factor Binding Protein-5, Interleukin-3, Interleukin-8, Midkine, Progesterone, Putrescine, Stem Cell Factor, TGF-alpha, Wntl, Wnt3a, Wnt5a, caspase-4, chemokine ligand 1, chemokine ligand 2, chemokine ligand 5, chemokine ligand 7, chemokine ligand 11, chemokine ligand 20, haptoglobin, lectin, cholesterol 25-hydroxylase, syntaxin-8, syntaxin-11, ceruloplasmin, complement component 1, complement component 3, integrin alpha 6, lysosomal acid lipase 1, β-2 microglobulin, ubiquitin, macrophage migration inhibitory factor, cofilin, cyclophillin A, FKBP12, NDPK, profilin 1, cystatin C, calcyclin, stanniocalcin-1, PGE-2, mpCCL2, IDO, iNOS, HLA-G5, M-CSF, PIGF, MCP-1, extracellular matrix molecules, CCL2 (MCP-1), CCL3 (MIP-1α), CCL4 (MIP-1β), CCL5 (RANTES), CCL7 (MCP-3), CCL20 (MIP-3α), CCL26 (eotaxin-3), CX3CL1 (fractalkine), CXCL5 (ENA-78), CXCL11 (i-TAC), CXCL1 (GROα), CXCL2 (GROβ), CXCL8 (IL-8), CCL10 (IP-10), and combinations thereof.

15. Process according to any one of claims 13 to 14 wherein said cardiogenic composition is diluted in a medium containing compounds selected from the group consisting of foetal calf serum, human serum, platelet lysate, platelet-derived growth factor, and mixtures thereof and compounds selected from:
- A first group consisting of: TGFβ-1, BMP-4, α-thrombin, a compound selected from the group consisting of Cardiotrophin 1 and IL-6, and a compound selected from the group consisting of Cardiogenol C and retinoic acid;
- A second group consisting of TGFβ-1, BMP-4, α-thrombin, Cardiotrophin 1, IL-6, retinoic acid and Cardiogenol C;
- A third group consisting of Activin-A, FGF-2, IL-6, IGF-1 and retinoic acid; and,
- A fourth group consisting of TGF-β1, TGF-β2, TNF-α, BMP-1, BMP-2, BMP-4, BMP-6, FGF-2, FGF-4, FGF-5, FGF-12, FGF-13, FGF-15, FGF-20, leukaemia inhibitory factor, VEGF-A, VEGF-C, insulin like growth factor 1, interleukin 6 (IL-6), Activin A, α-thrombin, Retinoic acid, Cardiotrophin 1, Cardiogenol C, and combinations thereof;

16. Process according to any one of claim 13 to 15, wherein when cells committed to the generation of heart tissue are cardiopoietic cells, identity of said cardiopoietic cells corresponds to an observed increase in expression level of at least one gene in the group consisting of Nkx2.5, Tbx5, MEF2C, GATA4, GATA6, Mesp1, FOG1, FOG2, Flk1 and homologues thereof.

17. Process according to claim 16 wherein the increase of aid gene expression is a minimum 2-fold as determined by qPCR method, as compared to a reference.

18. Process according to any one of claims 13 to 17 wherein when cells committed to the generation of heart tissue are cardiopoietic cells, identity of said cardiopoietic cells is considered fulfilled by the observed presence of at least one polypeptide species chosen from the group consisting of Nkx2.5, Tbx5, MEF2C, GATA4, GATA6, Mesp1, FOG1, FOG2, Flk1 and homologues thereof, as compared to a reference, and either Nkx2.5 or MEF2C or both being further translocated to the nuclei of the cardiopoietic cells.

19. Process according to any one of claims 13 to 18, wherein homogeneity is fulfilled when at least 50%, preferably at least 85%, in a given sample are cardiopoietic cells.

20. Kit for the administration of a pharmaceutical composition according to any one of claims 1 to 11 which comprises a container containing said pharmaceutical composition.

## Patentansprüche

1. Pharmazeutische Zusammensetzung, beinhaltend auf die Erzeugung von Herzgewebe festgelegte Zellen, wobei die Zellen Folgendes zeigen:
i. einen im Vergleich mit einer Referenz beobachteten Anstieg des Expressionslevels von MEF2C und optional mindestens einem weiteren Gen, ausgewählt aus der Gruppe, bestehend aus Nkx2.5, Tbx5, GATA4, GATA6, Mesp1, FOG1, FOG2, Flk1; und/oder
ii. ein im Vergleich mit einer Referenz beobachtetes Vorliegen von mindestens einer Polypeptidspezies, gewählt aus der Gruppe, bestehend aus Nkx2.5, Tbx5, GATA4, GATA6, Mesp1, FOG1, FOG2, Flk1, und nukleäre Translokation zu den Kernen der Zelle von Nkx2.5 und/oder MEF2C;
und mindestens einen pharmazeutisch akzeptablen Hilfsstoff, wobei der mindestens eine pharmazeutisch akzeptable Hilfsstoff eine Konservierungslösung ist.

2. Pharmazeutische Zusammensetzung gemäß Anspruch 1, wobei der beobachtete Anstieg des Expressionslevels laut der Bestimmung mittels qPCR im Vergleich mit einer Referenz mindestens zweifach ist.

3. Pharmazeutische Zusammensetzung gemäß einem der vorhergehenden Ansprüche, wobei die Konservierungslösung aus der Gruppe gewählt ist, die Konservierungslösungen, die eine Kryokonservierung bei Temperaturen zwischen -196 °C und 0 °C erlauben, und Konservierungslösungen, die eine Konservierung bei Temperaturen zwischen 0 °C und +40 °C erlauben, beinhaltet.

4. Pharmazeutische Zusammensetzung gemäß einem der vorhergehenden Ansprüche, wobei die Konservierungslösung eine Konservierungslösung ist, die Folgendes enthalten kann: Ionen (Na⁺, K⁺, Ca²⁺, Mg²⁺, Cl⁻ und Kombinationen davon), pH-Puffer (H₂PO₄⁻, HCO₃⁻, (4-(2-Hydroxyethyl)-1-piperazinethansulfonsäure) (HEPES) und Gemische davon), nicht membrangängige Stoffe (Lactobionat, Saccharose, Mannitol, Glucose und Kombinationen davon), Colloide (Dextran-40) und Metaboliten (Adenosin, Glutathion und Kombinationen davon), Dimethylsulfoxid (DMSO), Glycerol, Saccharose, Serumalbumin, Trehalose oder eine Kombination davon.

5. Pharmazeutische Zusammensetzung gemäß einem der vorhergehenden Ansprüche, wobei der mindestens eine pharmazeutisch akzeptable Hilfsstoff mindestens eine Komponente beinhaltet, ausgewählt aus der Gruppe, bestehend aus Wachstumsfaktoren, Zytokinen, an der Organgenese-Signalgebung beteiligten Proteinen, Pharmazeutika, Thrombozytenlysat, Serum, Isotopen, Mitteln zur In-vivo-Nachverfolgung von Zellen, Verdünnungsmitteln, Gleitmitteln, Matrix- oder Gerüstmaterialien und Kombinationen davon.

6. Pharmazeutische Zusammensetzung gemäß einem der vorhergehenden Ansprüche, wobei die auf die Erzeugung von Herzgewebe festgelegten Zellen Stammzellen sind (unter der Voraussetzung, dass ihre Produktion keine Vernichtung menschlicher Embryonen impliziert).

7. Pharmazeutische Zusammensetzung gemäß einem der vorhergehenden Ansprüche, wobei die auf die Erzeugung von Herzgewebe festgelegten Zellen aus Stammzellen gewonnen sind (unter der Voraussetzung, dass ihre Produktion keine Vernichtung menschlicher Embryonen impliziert).

8. Pharmazeutische Zusammensetzung gemäß Ansprüchen 6 oder 7, wobei die Stammzellen ausgewählt sind aus der Gruppe, bestehend aus adulten Stammzellen, einschließlich mesenchymaler Stammzellen; embryonaler Stammzellen (unter der Voraussetzung, dass ihre Produktion keine Vernichtung menschlicher Embryonen impliziert); induzierter pluripotenter Stammzellen (IPS); aus dem Knochenmark isolierter adulter zu mehreren Zellreihen induzierbarer Zellen (MIAMI); residenter kardialer Stammzellen; vegetaler Stammzellen; oder einer Kombination davon.

9. Pharmazeutische Zusammensetzung gemäß einem der vorhergehenden Ansprüche, wobei die auf die Erzeugung von Herzgewebe festgelegten Zellen Säugerzellen sind.

10. Pharmazeutische Zusammensetzung gemäß einem der vorhergehenden Ansprüche, wobei die auf die Erzeugung von Herzgewebe festgelegten Zellen kardiopoetische Zellen sind, die aus menschlichen adulten mesenchymalen Stammzellen, humanen embryonalen Stammzellen (unter der Voraussetzung, dass ihre Produktion keine Vernichtung menschlicher Embryonen impliziert), embryonalartigen Stammzellen, induzierbaren pluripotenten Stammzellen, kardial residenten Stammzellen oder einer anderen adaptierten Quelle oder Kombination davon gewonnen werden können.

11. Pharmazeutische Zusammensetzung gemäß Anspruch 10, wobei zwischen 0 % und 50 % der Gesamtzahl der Zellen, bevorzugt zwischen 0 % und 15 % der Gesamtzahl der Zellen nicht kardiopoetische Zellen sind, insbesondere Kardiomyozyten, hämatopoetische Zellen, Endothelvorläuferzellen, Adipoblasten, Adipozyten, Chondroblasten, Chondrozyten, Osteoblasten, Osteozyten, Neuroblasten und Neurozyten.

12. Pharmazeutische Zusammensetzung gemäß einem der Ansprüche 1 bis 11 zur Verwendung bei der Behandlung der ischämischen Kardiomyopathie, des akuten Myokardinfarkts, des chronischen Myokardinfarkts, der Herzinsuffizienz nicht ischämischen Ursprungs, der Herzinsuffizienz ischämischen Ursprungs, der kongenitalen Kardiomyopathie oder einer Kombination davon.

13. Prozess zur Herstellung der pharmazeutischen Zusammensetzung gemäß einem der vorhergehenden Ansprüche, der die folgenden Schritte beinhaltet:
- Erhalten von Zellen, aus denen auf die Erzeugung von Herzgewebe festgelegte Zellen gewonnen werden können;
- Kultivieren der Zellen unter Bedingungen, die es erlauben, auf die Erzeugung von Herzgewebe festgelegte Zellen zu erhalten;
- Ernten der festgelegten Zellen;
- Zugeben von mindestens einem pharmazeutisch akzeptablen Hilfsstoff zu den festgelegten Zellen, wobei der mindestens eine pharmazeutisch akzeptable Hilfsstoff eine Konservierungslösung ist.

14. Prozess gemäß Anspruch 13, wobei die Zellen, aus denen auf die Erzeugung von Herzgewebe festgelegte Zellen gewonnen werden können, in Kontakt mit einer kardiogenen Zusammensetzung gebracht werden, die aus einer oder mehreren kardiogenen Substanzen zusammengesetzt ist, gewählt aus der Gruppe, bestehend aus: Activin A, α-Thrombin, Angiopoetin, knochenmorphogenetischen Proteinen (BMP) wie etwa BMP-1, BMP-2, BMP-4, BMP-5, BMP-6, Cardiotrophin 1, Cardiogenol C, epidermalem Wachstumsfaktor (EGF), Erythropoetin (EPO), Fibroblasten-Wachstumsfaktoren (FGF) wie etwa FGF-1, FGF-2, FGF-4, FGF-5, FGF-12, FGF-13, FGF-15, FGF-20, granulozytenkoloniestimulierendem Faktor (G-CSF), granulozytenmakrophagenkoloniestimulierendem Faktor (GM-CSF), Wachstumsdifferenzierungsfaktor 9 (GDF-9), Hepatozyten-Wachstumsfaktor (HGF), insulinartigem Wachstumsfaktor (IGF) wie etwa IGF-1, IGF-2, Myostatin (GDF-8), Neurotrophinen wie etwa NT-3, NT-4, NT-1 und Nerven-Wachstumsfaktor (NGF), thrombozytenderiviertem Wachstumsfaktor (PDGF) wie etwa PDGF-beta, PDGF-AA, PDGF-BB, Thrombopoetin (TPO), transformierendem Wachstumsfaktor-α (TGF-α), transformierenden Wachstumsfaktoren β (TGF-β) wie etwa TGF-β1, TGF-β2, TGF-β3, VEGF (vaskulärem endothelialem Wachstumsfaktor) wie etwa VEGF-A, VEGF-C, TNF-α, leukämiehemmendem Faktor (LIF), Interleukin 6 (IL-6), Retinsäure, C SDF-1 (stromalem zellderiviertem Faktor-1), BDNF (gehirnderiviertem neurotrophem Faktor), Periostin, Angiotensin II, Flt3-Ligand, glialderiviertem neurotrophem Faktor, insulinartigem Wachstumsfaktor-Bindungsprotein 3, insulinartigem Wachstumsfaktor-Bindungsprotein 5, Interleukin-3, Interleukin-8, Midkin, Progesteron, Putrescin, Stammzellenfaktor, TGF-alpha, Wntl, Wnt3a, Wnt5a, Caspase-4, Chemokin-Ligand 1, Chemokin-Ligand 2, Chemokin-Ligand 5, Chemokin-Ligand 7, Chemokin-Ligand 11, Chemokin-Ligand 20, Haptoglobin, Lectin, Cholesterin-25-hydroxylase, Syntaxin-8, Syntaxin-11, Ceruloplasmin, Komplementkomponente 1, Komplementkomponente 3, Integrin-alpha-6, lysosomaler Säurelipase-1, β-2-Mikroglobulin, Ubiquitin, makrophagenmigrationshemmendem Faktor, Cofilin, Cyclophillin A, FKBP12, NDPK, Profilin 1, Cystatin C, Calcyclin, Stanniocalcin-1, PGE-2, mpCCL2, IDO, iNOS, HLA-G5, M-CSF, PIGF, MCP-1, extrazellulären Matrixmolekülen, CCL2 (MCP-1), CCL3 (MIP-1α), CCL4 (MIP-1β), CCL5 (RANTES), CCL7 (MCP-3), CCL20 (MIP-3α), CCL26 (Eotaxin-3), CX3CL1 (Fractalkin), CXCL5 (ENA-78), CXCL11 (i-TAC), CXCL1 (GROα), CXCL2 (GROβ), CXCL8 (IL-8), CCL10 (IP-10) und Kombinationen davon.

15. Prozess gemäß einem der Ansprüche 13 bis 14, wobei die kardiogene Zusammensetzung in einem Medium verdünnt ist, das Verbindungen enthält, ausgewählt aus der Gruppe, bestehend aus fetalem Kalbsserum, menschlichem Serum, Thrombozytenlysat, thrombozytenderiviertem Wachstumsfaktor und Gemischen davon und Verbindungen, ausgewählt aus:
- einer ersten Gruppe, bestehend aus: TGFβ-1, BMP-4, α-Thrombin, einer Verbindung, ausgewählt aus der Gruppe, bestehend aus Cardiotrophin 1 und IL-6, und einer Verbindung, ausgewählt aus der Gruppe, bestehend aus Cardiogenol C und Retinsäure;
- einer zweiten Gruppe, bestehend aus: TGFβ-1, BMP-4, α-Thrombin, Cardiotrophin 1, IL-6, Retinsäure und Cardiogenol C;
- einer dritten Gruppe, bestehend aus: Activin-A, FGF-2, IL-6, IGF-1 und Retinsäure;
- einer vierten Gruppe, bestehend aus: TGF-β1, TGF-β2, TNF-α, BMP-1, BMP-2, BMP-4, BMP-6, FGF-2, FGF-4, FGF-5, FGF-12, FGF-13, FGF-15, FGF-20, leukämiehemmendem Faktor, VEGF-A, VEGF-C, insulinartigem Wachstumsfaktor 1, Interleukin 6 (IL-6), Activin A, α-Thrombin, Retinsäure, Cardiotrophin 1, Cardiogenol C und Kombinationen davon.

16. Prozess gemäß einem der Ansprüche 13 bis 15, wobei, wenn die auf die Erzeugung von Herzgewebe festgelegten Zellen kardiopoetische Zellen sind, die Identität der kardiopoetischen Zellen einem beobachteten Anstieg des Expressionslevels von mindestens einem Gen in der Gruppe, bestehend aus Nkx2.5, Tbx5, MEF2C, GATA4, GATA6, Mesp1, FOG1, FOG2, Flk1 und Homologen davon, entspricht.

17. Prozess gemäß Anspruch 16, wobei der Anstieg der Genexpression laut der Bestimmung mittels qPCR im Vergleich mit einer Referenz mindestens zweifach ist.

18. Prozess gemäß einem der Ansprüche 13 bis 17, wobei, wenn die auf die Erzeugung von Herzgewebe festgelegten Zellen kardiopoetischen Zellen sind, die Identität der kardiopoetischen Zellen als erfüllt gilt, wenn das Vorliegen von mindestens einer Polypeptidspezies, gewählt aus der Gruppe, bestehend aus Nkx2.5, Tbx5, MEF2C, GATA4, GATA6, Mesp1, FOG1, FOG2, Flk1 und Homologen davon, im Vergleich mit einer Referenz beobachtet wird, und wenn entweder Nkx2.5 oder MEF2C oder beide ferner zu den Kernen der kardiopoetischen Zellen transloziert sind.

19. Prozess gemäß einem der Ansprüche 13 bis 18, wobei die Homogenität erfüllt ist, wenn mindestens 50 %, bevorzugt mindestens 85 % in einer gegebenen Probe kardiopoetische Zellen sind.

20. Kit zum Verabreichen der pharmazeutischen Zusammensetzung gemäß einem der Ansprüche 1 bis 11, der einen Behälter beinhaltet, der die pharmazeutische Zusammensetzung enthält.

## Revendications

1. Composition pharmaceutique comprenant des cellules impliquées dans la génération de tissu cardiaque, dans laquelle lesdites cellules présentent :
i. une augmentation observée du taux d'expression par rapport à une référence de MEF2C et facultativement au moins un gène également choisi dans le groupe constitué par Nkx2.5, Tbx5, GATA4, GATA6, Mesp1, FOG1, FOG2, Flk1 ; et/ou
ii. une présence observée d'au moins une espèce polypeptidique choisie dans le groupe constitué par Nkx2.5, Tbx5, GATA4, GATA6, Mesp1, FOG1, FOG2, Flk1, par rapport à une référence et la translocation nucléaire vers les noyaux de la cellule d'un ou plusieurs de Nkx2.5 et MEF2C ;
et au moins un excipient pharmaceutiquement acceptable, dans laquelle ledit au moins un excipient pharmaceutiquement acceptable est une solution de conservation.

2. Composition pharmaceutique selon la revendication 1, dans laquelle l'augmentation observée du taux d'expression est d'au moins deux fois, telle que déterminée par qPCR, par rapport à une référence.

3. Composition pharmaceutique selon l'une quelconque des revendications précédentes, dans laquelle la solution de conservation est choisie dans le groupe comprenant les solutions de conservation qui permettent une cryoconservation à des températures comprises entre -196 °C et 0 °C et les solutions de conservation qui permettent une conservation à des températures comprises entre 0 °C et +40 °C.

4. Composition pharmaceutique selon l'une quelconque des revendications précédentes, dans laquelle la solution de conservation est une solution de conservation qui peut contenir des ions (Na⁺, K⁺ Ca²⁺, Mg²⁺, Cl⁻ et leurs mélanges), des tampons pH (H₂PO4-, HCO₃⁻, acide 4-(2-hydroxyéthyl)-1-pipérazine-éthanesulfonique (HEPES) et leurs mélanges), des imperméants (lactobionate, saccharose, mannitol, glucose et leurs mélanges), des colloïdes (dextran 40) et des métabolites (adénosine, glutathione, et leurs mélanges), du diméthylsulfoxyde (DMSO), du glycérol, du saccharose, de la sérumalbumine, du tréhalose, ou leurs mélanges.

5. Composition pharmaceutique selon l'une quelconque des revendications précédentes, dans laquelle ledit au moins un excipient pharmaceutiquement acceptable comprend au moins un composant choisi dans le groupe constitué par les facteurs de croissance, les cytokines, les protéines impliquées dans la signalisation de l'organogenèse, les substances pharmaceutiques, le lysat plaquettaire, le sérum, les isotopes, les moyens de suivi des cellules *in vivo,* les diluants, les lubrifiants, les matériaux matriciels et supports, et leurs mélanges.

6. Composition pharmaceutique selon l'une quelconque des revendications précédentes, dans laquelle lesdites cellules impliquées dans la génération de tissu cardiaque sont des cellules souches (à condition que leur production n'implique pas la destruction d'embryons humains).

7. Composition pharmaceutique selon l'une quelconque des revendications précédentes, dans laquelle lesdites cellules impliquées dans la génération de tissu cardiaque sont issues de cellules souches (à condition que leur production n'implique pas la destruction d'embryons humains).

8. Composition pharmaceutique selon la revendication 6 ou 7, dans laquelle lesdites cellules souches sont choisies dans le groupe constitué par les cellules souches adultes, y compris les cellules souches mésenchymateuses ; les cellules souches embryonnaires (à condition que leur production n'implique pas la destruction d'embryons humains) ; les cellules souches pluripotentes induites (CSPi) ; les cellules multipotentielles inductibles isolées de la moelle adulte (MIAMI) ; les cellules souches cardiaques résidentes ; les cellules souches végétales ; et leurs mélanges.

9. Composition pharmaceutique selon l'une quelconque des revendications précédentes, dans laquelle lesdites cellules impliquées dans la génération de tissu cardiaque sont des cellules mammifères.

10. Composition pharmaceutique selon l'une quelconque des revendications précédentes, dans laquelle lesdites cellules impliquées dans la génération de tissu cardiaque sont des cellules cardiopoïétiques qui peuvent être issues de cellules souches mésenchymateuses adultes humaines, de cellules souches embryonnaires humaines (à condition que leur production n'implique pas la destruction d'embryons humains), de cellules souches à caractère embryonnaire, de cellules souches pluripotentes inductibles, de cellules souches cardiaques résidentes ou de toute autre origine appropriée, ou leurs mélanges.

11. Composition pharmaceutique selon la revendication 10, dans laquelle entre 0 % et 50 % du nombre total de cellules, de préférence entre 0 % et 15 % du nombre total de cellules sont des cellules non cardiopoïétiques, y compris, entre autres, des cardiomyocytes, des cellules hématopoïétiques, des cellules progénitrices endothéliales, des adipoblastes, des adipocytes, des chondroblastes, des chondrocytes, des ostéoblastes, des ostéocytes, des neuroblastes et des neurocytes.

12. Composition pharmaceutique selon l'une quelconque des revendications 1 à 11, pour une utilisation dans le traitement de la cardiomyopathie ischémique, de l'infarctus aigu du myocarde, de l'infarctus chronique du myocarde, l'insuffisance cardiaque d'origine non ischémique, l'insuffisance cardiaque d'origine ischémique, la cardiomyopathie congénitale, ou leurs mélanges.

13. Procédé de fabrication d'une composition pharmaceutique selon l'une quelconque des revendications précédentes, comprenant les étapes consistant à:
- obtenir des cellules à partir desquelles les cellules impliquées dans la génération de tissu cardiaque peuvent être issues ;
- cultiver lesdites cellules dans des conditions qui permettent d'obtenir des cellules impliquées dans la génération de tissu cardiaque ;
- collecter lesdites cellules impliquées ;
- ajouter auxdites cellules impliquées au moins un excipient pharmaceutiquement acceptable, dans lequel ledit au moins un excipient pharmaceutiquement acceptable est une solution de conservation.

14. Procédé selon la revendication 13, dans lequel lesdites cellules à partir desquelles les cellules impliquées dans la génération de tissu cardiaque peuvent être issues sont mises en contact avec une composition cardiogénique constituée d'une ou de plusieurs substances cardiogéniques choisie(s) dans le groupe constitué par : l'activine A, l'α-thrombine, l'angiopoïétine, les protéines morphogénétiques osseuses (BMP) telles que BMP-1, BMP-2, BMP-4, BMP-5, BMP-6, la cardiotrophine 1, le cardiogénol C, le facteur de croissance épidermique (EGF), l'érythropoïétine (EPO), les facteurs de croissance des fibroblastes (FGF) tels que FGF-1, FGF-2, FGF-4, FGF-5, FGF-12, FGF-13, FGF-15, FGF-20, le facteur de stimulation des colonies de granulocytes (G-CSF), le facteur de stimulation des colonies de granulocytes et de macrophages (GM-CSF), le facteur 9 de différenciation de croissance (GDF-9), le facteur de croissance des hépatocytes (HGF), les facteurs de croissance insulinomimétiques (IGF) tels qu'IGF-1, IGF-2, la myostatine (GDF-8), les neurotrophines telles que NT-3, NT-4, NT-1 et le facteur de croissance des nerfs (NGF), les facteurs de croissance dérivés des plaquettes (PDGF) tels que PDGF-bêta, PDGF-AA, PDGF-BB, la thrombopoïétine (TPO), le facteur de croissance transformant α (TGF-α), les facteurs de croissance transformant β (TGF-β) tels que TGF-β1, TGF-β2, TGF-β3, les facteurs de croissance de l'endothélium vasculaire (VEGF) tels que VEGF-A, VEGF-C, TNF-α, le facteur inhibiteur de la leucémie (LIF), l'interleukine-6 (IL-6), l'acide rétinoïque, le facteur 1 dérivé des cellules stromales (SDF-1), le facteur neurotrophique dérivé du cerveau (BDNF), la périostine, l'angiotensine II, le ligand Flt3, le facteur neurotrophique dérivé des cellules gliales, la protéine de liaison du facteur de croissance insulinomimétique de type 3, la protéine de liaison du facteur de croissance insulinomimétique de type 5, l'interleukine-3, l'interleukine-8, la midkine, la progestérone, la putrescine, le facteur cellule souche, TGF-alpha, Wntl, Wnt3a, Wnt5a, la caspase-4, le ligand 1 de chimiokine, le ligand 2 de chimiokine, le ligand 5 de chimiokine, le ligand 7 de chimiokine, le ligand 11 de chimiokine, le ligand 20 de chimiokine, l'haptoglobine, la lectine, le cholestérol 25-hydroxylase, la syntaxine-8, la syntaxine-11, la céruloplasmine, le composant du complément 1, le composant du complément 3, l'intégrine alpha 6, la lipase acide lysosomale 1, la microglobuline β-2, l'ubiquitine, le facteur inhibiteur de la migration des macrophages, la cofiline, la cyclophiline A, FKBP12, NDPK, la profiline 1, la cystatine C, la calcycline, la stanniocalcine-1, PGE-2, mpCCL2, IDO, iNOS, HLA-G5, M-CSF, PIGF, MCP-1, les molécules de la matrice extracellulaire, CCL2 (MCP-1), CCL3 (MIP-1α), CCL4 (MIP-1β), CCL5 (RANTES), CCL7 (MCP-3), CCL20 (MIP-3α), CCL26 (éotaxine-3) CX3CL1 (fractalkine), CXCL5 (ENA-78), CXCL11 (i-TAC), CXCL1 (GROα), CXCL2 (GROβ), CXCL8 (IL-8), CCL10 (IP-10), et leurs mélanges.

15. Procédé selon l'une quelconque des revendications 13 à 14, dans lequel ladite composition cardiogénique est diluée dans un milieu contenant des composés choisis dans le groupe constitué par le sérum foetal de veau, le sérum humain, le lysat plaquettaire, le facteur de croissance dérivé des plaquettes, et leurs mélanges, et des composés choisis parmi :
- un premier groupe constitué par: TGF-β1, BMP-4, l'α-thrombine, un composé choisi dans le groupe constitué par la cardiotrophine 1 et IL-6, et un composé choisi dans le groupe constitué par le cardiogénol C et l'acide rétinoïque ;
- un deuxième groupe constitué par TGF-β1, BMP-4, l'α-thrombine, la cardiotrophine 1, IL-6, l'acide rétinoïque et le cardiogénol C ;
- un troisième groupe constitué par l'activine A, FGF-2, IL-6, IGF-1 et l'acide rétinoïque ; et,
- un quatrième groupe constitué par TGF-β1, TGF-β2, TNF-α, BMP-1, BMP-2, BMP-4, BMP-6, FGF-2, FGF-4, FGF-5, FGF-12, FGF-13, FGF-15, FGF-20, le facteur inhibiteur de la leucémie, VEGF-A, VEGF-C, le facteur 1 de croissance insulinomimétique, l'interleukine-6 (IL-6), l'activine A, l'α-thrombine, l'acide rétinoïque, la cardiotrophine 1, le cardiogénol C, et leurs mélanges.

16. Procédé selon l'une quelconque des revendications 13 à 15, dans lequel lorsque les cellules impliquées dans la génération de tissu cardiaque sont des cellules cardiopoïétiques, l'identité desdites cellules cardiopoïétiques correspond à une augmentation observée du taux d'expression d'au moins un gène dans le groupe constitué par Nkx2.5, Tbx5, MEF2C, GATA4, GATA6, Mesp1, FOG1, FOG2, Flk1 et leurs homologues.

17. Procédé selon la revendication 16, dans lequel l'augmentation de ladite expression génétique est d'au moins deux fois, telle que déterminée par la méthode qPCR, par rapport à une référence.

18. Procédé selon l'une quelconque des revendications 13 à 17, dans lequel lorsque les cellules impliquées dans la génération de tissu cardiaque sont des cellules cardiopoïétiques, l'identité desdites cellules cardiopoïétiques est considérée conforme par la présence observée d'au moins une espèce polypeptidique choisie dans le groupe constitué par Nkx2.5, Tbx5, MEF2C, GATA4, GATA6, Mesp1, FOG1, FOG2, Flk1, et leurs homologues, par rapport à une référence, et soit Nkx2.5 soit MEF2C, ou les deux, sont également translocalisés vers les noyaux des cellules cardiopoïétiques.

19. Procédé selon l'une quelconque des revendications 13 à 18, dans lequel l'homogénéité est conforme lorsqu'au moins 50 %, de préférence au moins 85 %, dans un échantillon donné, sont des cellules cardiopoïétiques.

20. Kit d'administration d'une composition pharmaceutique selon l'une quelconque des revendications 1 à 11 qui comprend un récipient contenant ladite composition pharmaceutique.
